# EUROPEAN PATENT SPECIFICATION

(11) **EP 4 580 738 B1**
(45) Date of publication and mention of the grant of the patent: **02.09.2026**
(21) Application number: 23735343.8
(22) Date of filing: 03.07.2023
(51) Int. Cl.: A61N 1/05, A61N 1/36, H01R 43/027, A61N 1/375, H01R 4/50, H01R 4/60

(54) **METHOD FOR MANUFACTURING AN IMPLANTABLE LEAD AND IMPLANTABLE LEAD**
VERFAHREN ZUR HERSTELLUNG EINER IMPLANTIERBAREN LEITUNG UND IMPLANTIERBARE LEITUNG
PROCÉDÉ DE FABRICATION D'UN CONDUCTEUR IMPLANTABLE ET CONDUCTEUR IMPLANTABLE

(30) Priority: 02.09.2022 EP 22193588
(43) Date of publication of application: 09.07.2025
(73) Proprietor: BIOTRONIK SE & Co. KG, 12359 Berlin (DE)
(72) Inventor: ZIMPEL, Dominik, 13129 Berlin (DE); THÜRKOW, Andreas, 12107 Berlin (DE); FELDMANN, Jörg, 12683 Berlin (DE)
(74) Representative: Biotronik Corporate Services SE
(86) International application number: PCT/EP2023/068158
(87) International publication number: WO 2024/046624

(56) References cited:
- EP-B1- 2 789 367
- DE-A1- 102005 039 038
- US-A1- 2011 130 818
- US-B2- 7 890 184
- US-B2- 9 265 929

## Description

The present invention relates to a method for manufacturing an implantable lead and to an implantable lead.

Implantable leads with ring electrodes can be used in the context of implantable medical devices such as neurostimulators for pain management. Such surgical leads should be as thin as possible and also very flexible.

Usually, the ring electrodes are electrically and mechanically connected to a conductor by welding and/or crimping. However, this can be difficult, especially when very small ring electrodes are used.

DE 10 2005 039 038 A1 shows a medical electrode device comprising a coil-shaped conductor. A stripped portion of the conductor can be placed on an outer surface of an inner support sleeve sitting on top of the conductor. An outer sleeve can then be slid onto the inner sleeve so that the stripped portion is electrically and mechanically connected to both sleeves. EP 2 789 367 A1 shows another example of a ring electrode comprising a coil-shaped conductor and a combination of concentric sleeves for electrically contacting the conductor. DE 3140015 A1 shows a ring electrode for an implantable lead, which can be electrically and mechanically connected to a coil-shaped conductor by compressing the ring electrode radially by means of a die. US7890184 discloses a method for manufacturing an implantable lead as well as said lead.

It therefore may be seen as an objective of the present invention to provide an improved method for manufacturing an implantable lead. In particular, the objective may be to provide a method for manufacturing an implantable lead by which a conductor can be stripped and connected to an electrode in the same step and/or by which a conductor and an electrode can be connected to each other without welding and/or crimping.

A further objective of the invention may be to provide an improved implantable lead.

These objectives may be achieved by the subject-matter of the independent claims. Advantageous embodiments are defined in the dependent claims as well as in the corresponding specification and figures.

A first aspect of the invention relates to a method for manufacturing an implantable lead. The method comprises at least the following steps which may be performed, for example, in the following order: (i) providing an annular first electrode and an annular second electrode, a diameter of an inner surface of the first electrode being larger than a diameter of an outer surface of the second electrode and providing at least one wire comprising an electrically conductive core, the core being surrounded by an electrically insulating sheathing in a sheathed wire portion; (ii) aligning the first electrode relative to the second electrode so that their individual central axes form a common central axis; (iii) positioning at least a part of the sheathed wire portion opposite to the inner surface of the first electrode and/or opposite to the outer surface of the second electrode; and (iv) electrically and mechanically connecting the wire to the first electrode and the second electrode by pressing the first electrode and the second electrode together along the common central axis, wherein an axial force in a direction parallel to the common central axis is applied to the sheathed wire portion by the first electrode and the second electrode being pressed together so that at least a part of the sheathing is removed from the core to obtain an exposed wire portion, wherein at least a part of the exposed wire portion is inserted into an overlap area where the inner surface of the first electrode at least partially overlaps the outer surface of the second electrode and compressed in the overlap area by the first electrode and the second electrode.

By applying the axial force to the sheathed wire portion, the sheathing can be cut and/or torn locally and pushed away from the core to provide the exposed wire portion. Thus, the wire can be at least partially stripped and electrically and mechanically connected to the electrodes in the same step without having to perform any additional stripping, welding and/or crimping steps.

The dimensions of the electrodes with respect to each other and/or to the wire may be such that the resulting electrical and mechanical connection is sufficiently robust to withstand normal stresses encountered during and/or after implantation of the implantable lead in a patient.

This may help to significantly reduce manufacturing costs.

For example, two or more wires may be connected to the electrodes in this manner.

A second aspect of the invention relates to an implantable lead. The implantable lead comprises: an annular first electrode; an annular second electrode, a diameter of an inner surface of the first electrode being larger than a diameter of an outer surface of the second electrode; at least one wire comprising an electrically conductive core, the core being exposed in an exposed wire portion; wherein the first electrode and the second electrode are arranged concentrically to each other so that the inner surface of the first electrode at least partially overlaps the outer surface of the second electrode in an overlap area; wherein at least a part of the exposed wire portion is inserted into the overlap area and compressed therein by the first electrode and the second electrode; wherein a free end of the wire protrudes from the overlap area in a direction parallel to a common central axis of the first electrode and the second electrode.

**In** other words, the wire can be electrically and mechanically connected to both electrodes without having to weld and/or crimp them together. Nonetheless, the connection may be hermetically sealed and/or sufficiently robust to withstand normal stresses encountered during and/or after implantation of the implantable lead in a patient. Thus, the manufacturing costs for the implantable lead can be significantly reduced.

The implantable lead, which may also be called a surgical lead, may be part of an implantable neurostimulator such as, for example, a spinal cord stimulator.

For example, the implantable lead may have been manufactured with the method as described above and below. Thus, features of the implantable lead may be considered to be features of the method as described above and below, and *vice versa.*

Embodiments of the invention may be considered, without limiting the invention, as being based on the ideas and findings described below.

The first electrode and the second electrode may both be made of a biocompatible and electrically conductive material such as, for example, stainless steel, nickel alloy, e.g., MP35N, platinum or a metal comprising a platinum compound, e.g., platinum-iridium. The electrodes may be made of the same material or different materials.

Both electrodes may be closed, i.e. using non-slotted, rings or sleeves. It is, however, possible that the (smaller) second electrode is thinner than the first electrode. This may be to guarantee that only the second electrode is significantly deformed, i.e., compressed, when both electrodes are pressed together. Accordingly, the first electrode may have more or less the same outer diameter before and after pressing.

The core of the wire may be made of an electrically conductive material such as, for example, silver or MP35N. The core may be, for example, a rope comprising a plurality of strands (a wire having a core with multiple strands may also be called a stranded wire).

The sheathing may be made of an electrically insulating material such as, for example, ETFE or PTFE.

One or both of the electrodes may have defined peripheral edges and/or a defined surface roughness to cut and/or remove the sheathing in a controlled manner.

According to an embodiment, the method may further comprise: providing an elongated lead body and at least one electrically conductive contact element for contacting body tissue; electrically and mechanically connecting a free end of the wire to the contact element; attaching the contact element to a distal end of the lead body; attaching the second electrode, which is pressed together with the first electrode, to a proximal end of the lead body, for example, by inserting the lead body into a central opening of the second electrode. The lead body may be a rod or tube made of a biocompatible and electrically insulating material such as, for example, silicone. The contact element may be part of a contact paddle comprising a plurality of contact elements arranged in a specific pattern. Such an implantable lead may be used for neurostimulation, e.g., spinal cord stimulation.

According to an embodiment, the compressed part of the exposed wire portion may have a longitudinal axis parallel to the common central axis. This may help to avoid excessive bending of the free end of the wire in further manufacturing steps.

According to an embodiment, the core may be surrounded by an electrically insulating sheathing in a sheathed wire portion. At least a part of the sheathed wire portion may protrude as the free end of the wire from the overlap area.

According to an embodiment, a part of the sheathed wire portion may be inserted into the overlap area and compressed therein by the first electrode and the second electrode.

According to an embodiment, a first free end of the wire may protrude from the overlap area in a first direction parallel to the common central axis and a second free end of the wire may protrude from the overlap area in a second direction opposite to the first direction.

According to an embodiment, an outer diameter of the wire may be 0.5 mm or less, particularly 0.2 mm or less, more particularly 0.1 mm or less (comparable to the size of human hair). According to a preferred embodiment, the outer diameter of the wire is 0.14mm.

According to an embodiment, the diameter of the outer surface of the second electrode may be 3 mm or less, 2 mm or less, particularly 1 mm or less. Such annular electrodes can be provided, for example, by electrical discharge machining (EDM) and/or mechanical machining, e.g., turning.

According to an embodiment, the diameter of the outer surface of the second electrode may differ from the diameter of the inner surface of the first electrode by 10% or less, particularly by 5% or less. In general, the difference between the two diameters may be chosen in dependence of the diameter of the wire, more specifically of the exposed wire portion and/or the sheathed wire portion. The difference should be small enough to compress the wire between the two electrodes in such a way that the resulting frictional connection is strong enough to withstand the stresses encountered during normal use of the implantable lead.

According to an embodiment, the diameter of the outer surface of the second electrode may differ from the diameter of the inner surface of the first electrode by 0.5 mm or less, particularly by 0.2 mm or less, more particularly by 0.1 mm or less. Such value ranges have proven to be particularly suitable in tests.

According to an embodiment, the first electrode may have a greater wall thickness than the second electrode. This has the effect that the first electrode is more stable in a radial direction transverse to the common central axis than the second electrode. Thus, the first electrode may be deformed less than the second electrode and/or to a negligible degree when they are pressed together.

According to an embodiment, a wall thickness of the first electrode may differ from a wall thickness of the second electrode by 10% to 75%, particularly by 25%. Tests have shown that such differences are particularly suitable for avoiding excessive deformation of the first electrode during the pressing.

According to an embodiment, the first electrode and the second electrode may be made of the same material. This may help to further reduce the manufacturing costs.

According to an embodiment, the wire may be a stranded wire. In other words, the wire may comprise a plurality of individual wires which may form the core of the (stranded) wire. The individual wires may be twisted and/or braided together to form an electrically conductive rope. This makes the wire more robust and more flexible.

It has to be noted that possible features and advantages of embodiments of the invention are described above and below partly with reference to a method for assessing pain, partly with reference to a corresponding data processing device. A person skilled in the art will recognize that the features described for individual embodiments can be transferred, adapted and/or interchanged in an analogous and suitable manner to other embodiments in order to arrive at further embodiments of the invention and possibly synergistic effects.

Advantageous embodiments of the invention are further explained below with reference to the accompanying drawings. Neither the drawings nor the description are to be interpreted as limiting the invention.
Fig. 1 shows an implantable lead according to an embodiment of the invention.
Fig. 2 illustrates some steps of a method according to an embodiment of the invention.
Fig. 3 shows an electrode assembly resulting from the steps illustrated in fig. 2.
Fig. 4 shows an electrode assembly manufactured with a method according to an alternative embodiment of the invention.

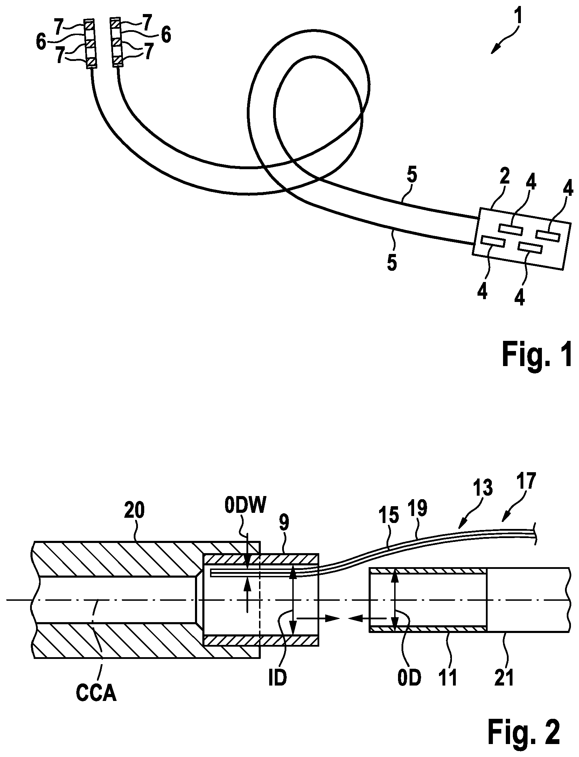

Fig. 1 shows an implantable lead 1 which may be part of an implantable neurostimulator such as, for example, a spinal cord stimulator. In this example, the implantable lead 1 comprises a contact paddle 2 on which a plurality of contact elements 4 for contacting body tissue, e.g., a portion of a spinal cord, are arranged in a specific pattern.

The contact paddle 2 may have two electrical poles. Each of the poles may be connected via an elongated lead body 5, which may be made of a biocompatible and electrically insulating material, e.g., in the form of a silicone tube, to a connector 6 at a proximal end of the implantable lead 1 (the contact paddle 2 may be attached to the lead bodies 5 at a distal end of the implantable lead 1). Each connector 6 may be connectable to a port of an implantable pulse generator of the neurostimulator.

For example, each connector 6 may comprise a plurality of electrode assemblies 7 for contacting the respective port. Each electrode assembly 7 may be connected via one or more wires (not shown in fig. 1), which may be integrated into the respective lead body 5, to one or more than one of the contact elements 4.

For example, the implantable lead 1 may have been manufactured with a method as described in more detail below referring to fig. 2, fig. 3 and fig. 4.

In a first step, an annular first electrode 9, an annular second electrode 11 and at least one wire 13 are provided. Both electrodes 9, 11 may have a cylindrical shape with an inner surface and an outer surface. An inner diameter ID of the inner surface of the first electrode 9 may be slightly larger than an outer diameter OD of the outer surface of the second electrode 11.

For example, the outer diameter OD may be 2 mm or less, particularly 1 mm or less, whereas the (larger) inner diameter ID may differ by 10% or less, particularly by 5% or less, from the outer diameter OD.

Depending on an outer diameter ODW of the wire 13, the outer diameter OD may be at most 0.5 mm smaller than the inner diameter ID. However, the difference may be even smaller, e.g., 0.2 mm or less or 0.1 mm or less.

The wire 13 comprises an electrically conductive core 15 which, in a sheathed wire portion 17, is surrounded by an electrically insulating sheathing 19. In this example, the sheathed wire portion 17 ends at a proximal end of the wire 13.

The outer diameter ODW may be at most 0.5 mm, particularly 0.2 mm or less or even 0.1 mm or less (comparable to the size of a human hair).

In a second step, the electrodes 9, 11 are centered so that they have a common central axis CCA. This may be done using a first electrode holder 20 that holds the first electrode 9 and a second electrode holder 21 that holds the second electrode 11. The electrode holders 20, 21 may be moveable relative to each other along the common central axis CCA.

**In** a third step, the sheathed wire portion 17 may be at least partially inserted into a central opening of the first electrode 9 so that at least a part of the sheathed wire portion 17 faces the inner surface of the first electrode 9.

**In** a fourth step, the two electrodes 9, 11 are pressed together by moving the electrode holders 20, 21 toward each other along the common central axis CCA, which may be done by moving either one or both of the electrode holders 20, 21. This has the effect that an axial force is applied in a direction parallel to the common central axis CCA by both electrodes 9, 11 to the sheathed wire portion 17, thereby locally cutting and/or tearing the sheathing 19 and pushing a part of it away from the proximal end of the core 15 to provide an exposed wire portion 23 where the core 15 is exposed, i.e., not surrounded by the sheathing 19. Thus, no further step may be required to strip the wire 13 at its proximal end.

Furthermore, by pressing the two electrodes 9, 11 together, the exposed wire portion 23 (or at least a part of it), which, in this example, ends at the proximal end of the wire 13, may be inserted into an overlap area 25 where the inner surface of the first electrode 9 partially or completely overlaps the outer surface of the second electrode 11. The inserted part of the exposed wire portion 23 may be compressed in the overlap area 25 by the two electrodes 9, 11 to form a robust electrical and mechanical connection. Thus, no further crimping and/or welding step may be required to electrically and/or mechanically connect the wire 13 to the two electrodes 9, 11.

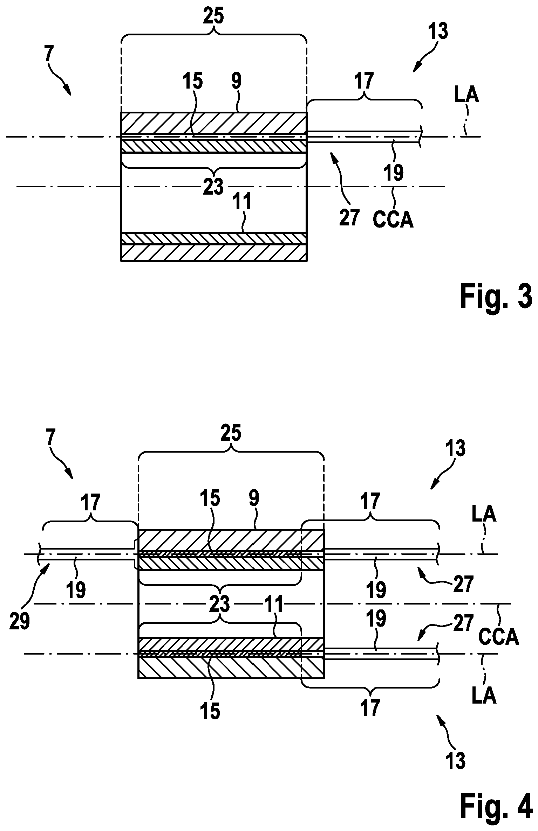

The resulting electrode assembly 7 is shown in fig. 3. As can be seen here, a free end 27 of the wire 13, which may comprise the exposed wire portion 23 and/or the sheathed wire portion 17, may protrude from the overlap area 25 in a direction parallel to the common central axis CCA.

In this example, the sheathed wire portion 17 protrudes as the free end 27 from the overlap area 25.

Additionally, the exposed wire portion 23 may have a longitudinal axis LA parallel to the common central axis CCA. Thus, the exposed wire portion 23 and the free end 27 may have a common longitudinal axis LA parallel to the common central axis CCA.

This may avoid excessive bending of the free end 27 in further manufacturing steps.

Furthermore, it is possible that one part of the sheathed wire portion 17 protrudes from the overlap area 25 and another part of the sheathed wire portion 17 is inserted into the overlap area 25 and compressed therein in the same way as the exposed wire portion 23. This may hermetically seal the connection (at least on one side of the electrode assembly 7).

As shown in fig. 4, it may be that more than one wire 13 is connected to both electrodes 9, 11.

Fig. 4 also shows that the (same) wire 13 may have a first free end 27 and a second free end 29 that protrude from opposite sides of the overlap area 25 (in this example, parts of the sheathed wire portion 17 protrude as the free ends 27, 29 from the overlap area 25).

For example, the free ends 27, 29 may protrude in opposite directions along the longitudinal axis LA, thus parallel to the common central axis CCA, to avoid excessive bending of the wire 13 in further manufacturing steps.

In additional steps, the electrode assembly 7 may be attached to a proximal end of one of the lead bodies 5, for example, by inserting the proximal end into a central opening of the second electrode 11. Furthermore, the free end of the wire 13 may be electrically and mechanically connected to the contact element(s) 4, which may be attached to a distal end of the respective lead body 5, i.e., the contact paddle 2.

It is possible that both electrodes 9, 11 are made of the same material, which may be a biocompatible metal.

However, the first electrode 9 may have a thicker wall than the second electrode 11 to avoid excessive deforming of the first electrode 9 when both electrodes 9, 11 are pressed together. For example, the wall thickness of the first electrode 9 may differ by 10% to 35%, particularly by 25%, from the wall thickness of the second electrode 11.

It has to be noted that, in the claims, the word "comprising" does not exclude other elements or steps, and the indefinite article "a" or "an" does not exclude a plurality. A single processor or controller or other unit may fulfil the functions of several items recited in the claims. The mere fact that certain measures are recited in mutually different dependent claims does not indicate that a combination of these measures cannot be used to advantage. Any reference signs in the claims should not be construed as limiting the scope of the claims.

It will be apparent to those skilled in the art that numerous modifications and variations of the described examples and embodiments are possible in light of the above teaching. The disclosed examples and embodiments are presented for purposes of illustration only. Other alternate embodiments may include some or all of the features disclosed herein.

## Claims

1. A method for manufacturing an implantable lead (1), the method comprising:
providing an annular first electrode (9) and an annular second electrode (11), a diameter (ID) of an inner surface of the first electrode (9) being larger than a diameter (OD) of an outer surface of the second electrode (11);
providing at least one wire (13) comprising an electrically conductive core (15), the core (15) being surrounded by an electrically insulating sheathing (19) in a sheathed wire portion (17);
aligning the first electrode (9) relative to the second electrode (11) so that their individual central axes form a common central axis (CCA);
positioning at least a part of the sheathed wire portion (17) opposite to the inner surface of the first electrode (9) and/or opposite to the outer surface of the second electrode (11); and
electrically and mechanically connecting the wire (13) to the first electrode (9) and the second electrode (11) by pressing the first electrode (9) and the second electrode (11) together along the common central axis (CCA),
wherein an axial force in a direction parallel to the common central axis (CCA) is applied to the sheathed wire portion (17) by the first electrode (9) and the second electrode (11) being pressed together so that at least a part of the sheathing (19) is removed from the core (15) to obtain an exposed wire portion (23),
wherein at least a part of the exposed wire portion (23) is inserted into an overlap area (25) where the inner surface of the first electrode (9) at least partially overlaps the outer surface of the second electrode (11) and compressed in the overlap area (25) by the first electrode (9) and the second electrode (11);
wherein the method further comprises:
providing an elongated lead body (5) and at least one electrically conductive contact element (4) for contacting body tissue;
electrically and mechanically connecting a free end of the wire (13) to the contact element (4);
attaching the contact element (4) to a distal end of the lead body (5);
attaching the second electrode (11), which is pressed together with the first electrode (9), to a proximal end of the lead body (5).

2. An implantable lead (1), comprising:
an annular first electrode (9);
an annular second electrode (11), a diameter (ID) of an inner surface of the first electrode (9) being larger than a diameter (OD) of an outer surface of the second electrode (11);
at least one wire (13) comprising an electrically conductive core (15), the core (15) being exposed in an exposed wire portion (23);
wherein the first electrode (9) and the second electrode (11) are arranged concentrically to each other so that the inner surface of the first electrode (9) at least partially overlaps the outer surface of the second electrode (11) in an overlap area (25);
wherein at least a part of the exposed wire portion (23) is inserted into the overlap area (25) and compressed therein by the first electrode (9) and the second electrode (11);
wherein a free end (27, 29) of the wire (13) protrudes from the overlap area (25) in a direction parallel to a common central axis (CCA) of the first electrode (9) and the second electrode (11);
wherein the implantable lead further comprises an elongated lead body (5) and at least one electrically conductive contact element (4) for contacting body tissue;
wherein a free end of the wire (13) is electrically and mechanically connected to the contact element (4);
wherein the contact element (4) is attached to a distal end of the lead body (5); and
wherein the second electrode (11), which is pressed together with the first electrode (9), is attached to a proximal end of the lead body (5).

3. The lead (1) of claim 2, wherein the compressed part of the exposed wire portion (23) has a longitudinal axis (LA) parallel to the common central axis (CCA).

4. The lead (1) of claim 3 or 2,
wherein the core (15) is surrounded by an electrically insulating sheathing (19) in a sheathed wire portion (17);
wherein at least a part of the sheathed wire portion (17) protrudes as the free end (27, 29) of the wire (13) from the overlap area (25).

5. The lead (1) of claim 4, wherein a part of the sheathed wire portion (17) is inserted into the overlap area (25) and compressed therein by the first electrode (9) and the second electrode (11).

6. The lead (1) of one of claims 2 to 5, wherein a first free end (27) of the wire (13) protrudes from the overlap area (25) in a first direction parallel to the common central axis (CCA) and a second free end (29) of the wire (13) protrudes from the overlap area (25) in a second direction opposite to the first direction.

7. The lead (1) of one of claims 2 to 6, wherein an outer diameter (ODW) of the wire (13) is 0.5 mm or less, particularly 0.2 mm or less, more particularly 0.1 mm or less.

8. The lead (1) of one of claims 2 to 7, wherein the diameter (OD) of the outer surface of the second electrode (11) is 3 mm or less, particularly 2 mm or less, particularly 1 mm or less.

9. The lead (1) of one of claims 2 to 8, wherein the diameter (OD) of the outer surface of the second electrode (11) differs from the diameter (ID) of the inner surface of the first electrode (9) by 10% or less, particularly by 5% or less.

10. The lead (1) of one of claims 2 to 9, wherein the diameter (OD) of the outer surface of the second electrode (11) differs from the diameter (ID) of the inner surface of the first electrode (9) by 0.5 mm or less, particularly by 0.2 mm or less, more particularly by 0.1 mm or less.

11. The lead (1) of one of claims 2 to 10, wherein the first electrode (9) has a greater wall thickness than the second electrode (11).

12. The lead (1) of one of claims 2 to 11, wherein a wall thickness of the first electrode (9) differs from a wall thickness of the second electrode (11) by 10% to 35%.

13. The lead (1) of one of claims 2 to 12, wherein the first electrode (9) and the second electrode (11) are made of the same material.

14. The lead (1) of one of claims 2 to 13, wherein the wire (13) is a stranded wire.

## Patentansprüche

1. Verfahren zur Herstellung einer implantierbaren Leitung (1), wobei das Verfahren umfasst:
Bereitstellen einer ringförmigen ersten Elektrode (9) und einer ringförmigen zweiten Elektrode (11), wobei der Durchmesser (ID) der Innenfläche der ersten Elektrode (9) größer ist als der Durchmesser (OD) der Außenfläche der zweiten Elektrode (11);
Bereitstellen mindestens eines Drahtes (13) mit einem elektrisch leitfähigen Kern (15), wobei der Kern (15) in einem ummantelten Drahtabschnitt (17) von einer elektrisch isolierenden Ummantelung (19) umgeben ist;
Ausrichten der ersten Elektrode (9) relativ zur zweiten Elektrode (11), so dass ihre jeweiligen Mittelachsen eine gemeinsame Mittelachse (CCA) bilden;
Positionieren mindestens eines Teils des ummantelten Drahtabschnitts (17) gegenüber der Innenfläche der ersten Elektrode (9) und/oder gegenüber der Außenfläche der zweiten Elektrode (11); und
elektrisches und mechanisches Verbinden des Drahtes (13) mit der ersten Elektrode (9) und der zweiten Elektrode (11) durch Zusammendrücken der ersten Elektrode (9) und der zweiten Elektrode (11) entlang der gemeinsamen Mittelachse (CCA),
wobei eine Axialkraft in einer Richtung parallel zur gemeinsamen Mittelachse (CCA) auf den ummantelten Drahtabschnitt (17) durch die erste Elektrode (9) und die zweite Elektrode (11) ausgeübt wird, die zusammengedrückt werden, so dass mindestens ein Teil der Ummantelung (19) vom Kern (15) entfernt wird, um einen freiliegenden Drahtabschnitt (23) zu erhalten,
wobei mindestens ein Teil des freiliegenden Drahtabschnitts (23) in einen Überlappungsbereich (25) eingeführt wird, in dem die Innenfläche der ersten Elektrode (9) mindestens teilweise die Außenfläche der zweiten Elektrode (11) überlappt, und in dem Überlappungsbereich (25) durch die erste Elektrode (9) und die zweite Elektrode (11) zusammengedrückt wird;
wobei das Verfahren ferner umfasst:
Bereitstellen eines länglichen Leitungskörpers (5) und mindestens eines elektrisch leitfähigen Kontaktelements (4) zum Kontaktieren von Körpergewebe;
elektrisches und mechanisches Verbinden eines freien Endes des Drahtes (13) mit dem Kontaktelement (4);
Befestigen des Kontaktelements (4) an einem distalen Ende des Leitungskörpers (5); Befestigen der zweiten Elektrode (11), die mit der ersten Elektrode (9) zusammengedrückt wird, an einem proximalen Ende des Leitungskörpers (5).

2. Implantierbare Leitung (1), umfassend:
eine ringförmige erste Elektrode (9);
eine ringförmige zweite Elektrode (11), wobei der Durchmesser (ID) der Innenfläche der ersten Elektrode (9) größer ist als der Durchmesser (OD) der Außenfläche der zweiten Elektrode (11);
mindestens einen Draht (13) mit einem elektrisch leitfähigen Kern (15), wobei der Kern (15) in einem freiliegenden Drahtabschnitt (23) freiliegt;
wobei die erste Elektrode (9) und die zweite Elektrode (11) konzentrisch zueinander angeordnet sind, so dass die Innenfläche der ersten Elektrode (9) die Außenfläche der zweiten Elektrode (11) in einem Überlappungsbereich (25) zumindest teilweise überlappt;
wobei mindestens ein Teil des freiliegenden Drahtabschnitts (23) in den Überlappungsbereich (25) eingeführt und dort durch die erste Elektrode (9) und die zweite Elektrode (11) zusammengedrückt wird;
wobei ein freies Ende (27, 29) des Drahtes (13) aus dem Überlappungsbereich (25) in einer Richtung parallel zu einer gemeinsamen Mittelachse (CCA) der ersten Elektrode (9) und der zweiten Elektrode (11) vorsteht;
wobei die implantierbare Leitung ferner einen länglichen Leitungskörper (5) und mindestens ein elektrisch leitfähiges Kontaktelement (4) zum Kontaktieren von Körpergewebe umfasst;
wobei ein freies Ende des Drahtes (13) elektrisch und mechanisch mit dem Kontaktelement (4) verbunden ist;
wobei das Kontaktelement (4) an einem distalen Ende des Leitungskörpers (5) befestigt ist; und
wobei die zweite Elektrode (11), die mit der ersten Elektrode (9) zusammengedrückt ist, an einem proximalen Ende des Leitungskörpers (5) befestigt ist.

3. Die Leitung (1) nach Anspruch 2, wobei der zusammengedrückte Teil des freiliegenden Drahtabschnitts (23) eine Längsachse (LA) parallel zur gemeinsamen Mittelachse (CCA) aufweist.

4. Die Leitung (1) nach Anspruch 3 oder 2,
wobei der Kern (15) von einer elektrisch isolierenden Ummantelung (19) in einem ummantelten Drahtabschnitt (17) umgeben ist;
wobei mindestens ein Teil des ummantelten Drahtabschnitts (17) als freies Ende (27, 29) des Drahtes (13) aus dem Überlappungsbereich (25) vorsteht.

5. Die Leitung (1) nach Anspruch 4, wobei ein Teil des ummantelten Drahtabschnitts (17) in den Überlappungsbereich (25) eingeführt und dort durch die erste Elektrode (9) und die zweite Elektrode (11) zusammengedrückt wird.

6. Die Leitung (1) nach einem der Ansprüche 2 bis 5, wobei ein erstes freies Ende (27) des Drahtes (13) aus dem Überlappungsbereich (25) in einer ersten Richtung parallel zur gemeinsamen Mittelachse (CCA) vorsteht und ein zweites freies Ende (29) des Drahtes (13) aus dem Überlappungsbereich (25) in einer zweiten Richtung entgegengesetzt zur ersten Richtung vorsteht.

7. Die Leitung (1) gemäß einem der Ansprüche 2 bis 6, wobei der Außendurchmesser (ODW) des Drahtes (13) 0,5 mm oder weniger, insbesondere 0,2 mm oder weniger, insbesondere 0,1 mm oder weniger beträgt.

8. Die Leitung (1) nach einem der Ansprüche 2 bis 7, wobei der Durchmesser (OD) der Außenfläche der zweiten Elektrode (11) 3 mm oder weniger, insbesondere 2 mm oder weniger, insbesondere 1 mm oder weniger beträgt.

9. Die Leitung (1) nach einem der Ansprüche 2 bis 8, wobei der Durchmesser (OD) der Außenfläche der zweiten Elektrode (11) um 10% oder weniger, insbesondere um 5% oder weniger, vom Durchmesser (ID) der Innenfläche der ersten Elektrode (9) abweicht.

10. Die Leitung (1) nach einem der Ansprüche 2 bis 9, wobei der Durchmesser (OD) der Außenfläche der zweiten Elektrode (11) um 0,5 mm oder weniger, insbesondere um 0,2 mm oder weniger, insbesondere um 0,1 mm oder weniger, vom Durchmesser (ID) der Innenfläche der ersten Elektrode (9) abweicht.

11. Die Leitung (1) nach einem der Ansprüche 2 bis 10, wobei die erste Elektrode (9) eine größere Wandstärke als die zweite Elektrode (11) aufweist.

12. Die Leitung (1) nach einem der Ansprüche 2 bis 11, wobei sich die Wandstärke der ersten Elektrode (9) um 10% bis 35% von der Wandstärke der zweiten Elektrode (11) unterscheidet.

13. Die Leitung (1) nach einem der Ansprüche 2 bis 12, wobei die erste Elektrode (9) und die zweite Elektrode (11) aus dem gleichen Material bestehen.

14. Die Leitung (1) eines der Ansprüche 2 bis 13, wobei der Draht (13) ein Litzendraht ist.

## Revendications

1. Procédé de fabrication d'un câble implantable (1), le procédé comprenant les étapes consistant à :
fournir une première électrode annulaire (9) et une seconde électrode annulaire (11), un diamètre (ID) d'une surface interne de la première électrode (9) étant supérieur à un diamètre (OD) d'une surface externe de la seconde électrode (11) ;
fournir au moins un fil (13) comprenant une âme électriquement conductrice (15), l'âme (15) étant entourée d'une gaine électriquement isolante (19) dans une portion de fil gainée (17) ;
aligner la première électrode (9) par rapport à la seconde électrode (11) de telle sorte que leurs axes centraux individuels forment un axe central commun (CCA) ;
positionner au moins une partie de la portion de fil gainée (17) de manière opposée à la surface interne de la première électrode (9) et/ou opposée à la surface externe de la seconde électrode (11) ; et
connecter électriquement et mécaniquement le fil (13) à la première électrode (9) et à la seconde électrode (11) en pressant la première électrode (9) et la seconde électrode (11) l'une contre l'autre le long de l'axe central commun (CCA),
dans lequel une force axiale dans une direction parallèle à l'axe central commun (CCA) est appliquée à la portion de fil gainée (17) par le fait de presser la première électrode (9) et la seconde électrode (11) l'une contre l'autre de telle sorte qu'au moins une partie de la gaine (19) est retirée de l'âme (15) pour obtenir une portion de fil exposée (23),
dans lequel au moins une partie de la portion de fil exposée (23) est insérée dans une zone de superposition (25) où la surface interne de la première électrode (9) se superpose au moins partiellement à la surface externe de la seconde électrode (11) et est comprimée dans la zone de superposition (25) par la première électrode (9) et la seconde électrode (11) ;
dans lequel le procédé comprend en outre les étapes consistant à :
fournir un corps de câble allongé (5) et au moins un élément de contact électriquement conducteur (4) pour entrer en contact avec un tissu corporel ;
connecter électriquement et mécaniquement une extrémité libre du fil (13) à l'élément de contact (4) ;
attacher l'élément de contact (4) à une extrémité distale du corps de câble (5) ;
attacher la seconde électrode (11), qui est pressée contre la première électrode (9), à une extrémité proximale du corps de câble (5).

2. Câble implantable (1), comprenant :
une première électrode annulaire (9) ;
une seconde électrode annulaire (11), un diamètre (ID) d'une surface interne de la première électrode (9) étant supérieur à un diamètre (OD)d'une surface externe de la seconde électrode (11) ;
au moins un fil (13) comprenant une âme électriquement conductrice (15), l'âme (15) étant exposée dans une portion de fil exposée (23) ;
dans lequel la première électrode (9) et la seconde électrode (11) sont agencées de manière concentrique l'une par rapport à l'autre de telle sorte que la surface interne de la première électrode (9) se superpose au moins partiellement à la surface externe de la seconde électrode (11) dans une zone de superposition (25) ;
dans lequel au moins une partie de la portion de fil exposée (23) est insérée dans la zone de superposition (25) et comprimée à l'intérieur de celle-ci par la première électrode(9) et la seconde électrode (11) ;
dans lequel une extrémité libre (27, 29) du fil (13) fait saillie depuis la zone de superposition (25) dans une direction parallèle à un axe central commun (CCA) de la première électrode (9) et de la seconde électrode (11) ;
dans lequel le câble implantable comprend en outre un corps de câble allongé (5) et au moins un élément de contact électriquement conducteur (4) pour entrer en contact avec un tissu corporel ;
dans lequel une extrémité libre du fil (13) est électriquement et mécaniquement connectée à l'élément de contact (4) ;
dans lequel l'élément de contact (4) est attaché à une extrémité distale du corps de câble (5) ; et
dans lequel la seconde électrode (11), qui est pressée contre la première électrode (9), est attachée à une extrémité proximale du corps de câble (5).

3. Câble (1) selon la revendication 2, dans lequel la partie comprimée de la portion de fil exposée (23) a un axe longitudinal (LA) parallèle à l'axe central commun (CCA).

4. Câble (1) selon la revendication 3 ou 2,
dans lequel l'âme (15) est entourée par une gaine électriquement isolante (19) dans une portion de fil gainée (17) ;
dans lequel au moins une partie de la portion de fil gainée (17) fait saillie en tant qu'extrémité libre (27, 29) du fil (13) depuis la zone de superposition (25).

5. Câble (1) selon la revendication 4, dans lequel une partie de la portion de fil gainée (17) est insérée dans la zone de superposition (25) et comprimée à l'intérieur de celle-ci par la première électrode(9) et la seconde électrode (11).

6. Câble (1) selon l'une des revendications 2 à 5, dans lequel une première extrémité libre (27) du fil (13) fait saillie depuis la zone de superposition (25) dans une première direction parallèle à l'axe central commun (CCA) et une seconde extrémité libre (29) du fil (13) fait saillie depuis la zone de superposition (25) dans une seconde direction opposée à la première direction.

7. Câble (1) selon l'une des revendications 2 à 6, dans lequel un diamètre externe (ODW) du fil (13) est de 0,5 mm ou moins, en particulier de 0,2 mm ou moins, plus particulièrement de 0,1 mm ou moins.

8. Câble (1) selon l'une des revendications 2 à 7, dans lequel le diamètre (OD) de la surface externe de la seconde électrode (11) est de 3 mm ou moins, en particulier de 2 mm ou moins, plus particulièrement de 1 mm ou moins.

9. Câble (1) selon l'une des revendications 2 à 8, dans lequel le diamètre (OD) de la surface externe de la seconde électrode (11) diffère du diamètre (ID) de la surface interne de la première électrode (9) de 10 % ou moins, en particulier de 5 % ou moins.

10. Câble (1) selon l'une des revendications 2 à 9, dans lequel le diamètre (OD) de la surface externe de la seconde électrode (11) diffère du diamètre (ID) de la surface interne de la première électrode (9) de 0,5 mm ou moins, en particulier de 0,2 mm ou moins, plus particulièrement de 0,1 mm ou moins.

11. Câble (1) selon l'une des revendications 2 à 10, dans lequel la première électrode (9) a une épaisseur de paroi supérieure à celle de la seconde électrode (11).

12. Câble (1) selon l'une des revendications 2 à 11, dans lequel une épaisseur de paroi de la première électrode (9) diffère d'une épaisseur de paroi de la seconde électrode (11) de 10 % à 35 %.

13. Câble (1) selon l'une des revendications 2 à 12, dans lequel la première électrode (9) et la seconde électrode (11) sont fabriquées en le même matériau.

14. Câble (1) selon l'une des revendications 2 à 13, dans lequel le fil (13) est un fil multibrin.
